(19) Europäisches Patentamt
European Patent Office
Office européen des brevets

(11) EP 0 718 261 A1

(12) EUROPÄISCHE PATENTANMELDUNG

(43) Veröffentlichungstag: **26.06.1996 Patentblatt 1996/26**

(51) Int. Cl.[6]: **C07C 17/12**, C07C 25/13

(21) Anmeldenummer: **95119332.5**

(22) Anmeldetag: **07.12.1995**

(84) Benannte Vertragsstaaten: **CH DE ES FR GB IE IT LI PT**

(30) Priorität: **20.12.1994 DE 4445548**

(71) Anmelder: **BAYER AG**
**51368 Leverkusen (DE)**

(72) Erfinder:
- **Mais, Franz-Josef, Dr.**
  **D-40591 Düsseldorf (DE)**
- **Bussmann, Werner, Dr.**
  **D-51373 Leverkusen (DE)**
- **Fiege, Helmut, Dr.**
  **D-51373 Leverkusen (DE)**

# (54) Verfahren zur Herstellung von 3-Fluor-4,6-dichlortoluol

(57) 3-Fluor-4,6-dichlortoluol kann auf besonders günstige Weise hergestellt werden, wenn man 3-Fluortoluol in Gegenwart eines Friedel-Crafts-Katalysators und eines heterocyclischen Co-Katalysators zunächst bei niedriger Temperatur chloriert und ein Gemisch erhält, das 3-Fluor-4-chlorbenzol und 3-Fluor-6-chlorbenzol enthält, und anschließend dieses Gemisch ohne Zwischenisolierung nach Zusatz von weiterem Friedel-Crafts-Katalysator und weiterem heterocyclischen Co-Katalysator bei höherer Temperatur zu 3-Fluor-4,6-dichlortoluol weiterchloriert.

EP 0 718 261 A1

## Beschreibung

Die vorliegende Erfindung betrifft ein verbessertes Verfahren zur Herstellung von 3-Fluor-4,6-dichlortoluol durch selektive Chlorierung von 3-Fluortoluol mit verminderter Bildung des Nebenprodukts 3-Fluor-2,6-dichlortoluol.

3-Fluor-4,6-dichlortoluol ist ein wichtiges Zwischenprodukt für die Herstellung von pharmazeutischen Wirkstoffen des Chinoloncarbonsäuretyps.

Für die Herstellung von 3-Fluor-4,6-dichlortoluol sind eine Reihe von Synthesewegen beschrieben, die durch ihre Vielstufigkeit besonderen Aufwand erfordern und/oder bei denen mit technisch nur schwer handhabbaren Stoffen umgegangen werden muß.

Es ist bekannt, 3-Fluor-4,6-dichlortoluol aus 3-Amino-4,6-dichlortoluol über die Stufen Diazotierung, Kupplung mit Dimethylamin, Umsetzung mit Fluorwasserstoff und thermische Spaltung herzustellen (siehe DE-OS 3 142 856). Dabei erhält man im letzten Schritt zwar gute Ausbeuten, die Herstellung umfaßt jedoch 4 Reaktionsstufen und die Handhabung einer leicht zersetzlichen Triazenverbindung.

Es wurde nun ein Verfahren zur Herstellung von 3-Fluor-4,6-dichlortoluol gefunden, das dadurch gekennzeichnet ist, daß man 3-Fluortoluol in Gegenwart eines Friedel-Crafts-Katalysators und eines heterocyclischen Co-Katalysators zunächst bei niedriger Temperatur chloriert und ein Gemisch erhält, das 3-Fluor-4-chlorbenzol und 3-Fluor-6-chlorbenzol enthält, und anschließend dieses Gemisch ohne Zwischenisolierung nach Zusatz von weiterem Friedel-Crafts-Katalysator und weiterem heterocyclischen Co-Katalysator bei höherer Temperatur zu 3-Fluor-4,6-dichlortoluol weiterchloriert.

Als Friedel-Crafts-Katalysatoren für beide Reaktionsstufen des erfindungsgemäßen Verfahrens kommen z.B. Metall- und Übergangsmetall-Halogenide in Frage. Bevorzugt sind Eisen(III)-chlorid, Antimon(III)-chlorid, Antimon(V)-chlorid und Aluminiumchlorid, besonders bevorzugt ist Eisen(III)-chlorid. In beide Reaktionsstufen können gleiche oder verschiedene Friedel-Crafts-Katalysatoren eingesetzt werden. Vorzugsweise wendet man in beiden Reaktionsstufen den gleichen Friedel-Crafts-Katalysator an.

In der ersten Reaktionsstufe kann man beispielsweise 0,05 bis 1 Gew.-% Friedel-Crafts-Katalysator (bezogen auf 3-Fluortoluol) einsetzen. Vorzugsweise beträgt diese Menge 0,1 bis 1 Gew.-%. In die zweite Reaktionsstufe kann man beispielsweise weitere 0,1 bis 5 Gew.-% Friedel-Crafts-Katalysator (bezogen auf 3-Fluortoluol) zufügen. Vorzugsweise beträgt diese zusätzliche Menge 0,5 bis 2 Gew.-%.

Als Co-Katalysatoren für beide Reaktionsstufen des erfindungsgemäßen Verfahrens können z.B. Heterocyclen eingesetzt werden, die gleichzeitig N- und S-Atome enthalten und z.B. den Klassen der Thiazine, Thiazepine oder Thiazocine angehören. Beispielsweise seien genannt:
Thiazepine der Formeln

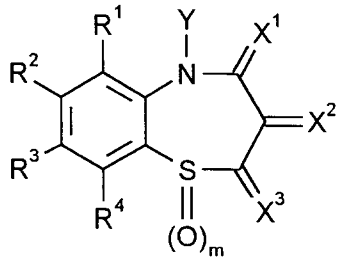

(I),

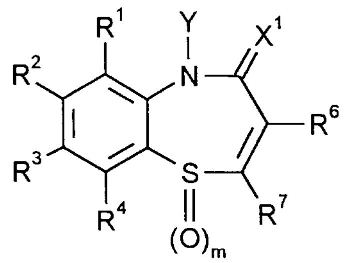

(II),

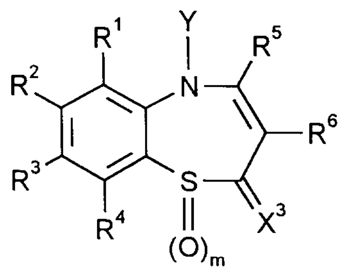

(III),

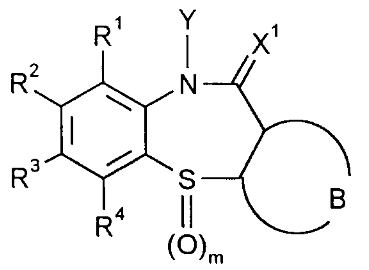

(IV),

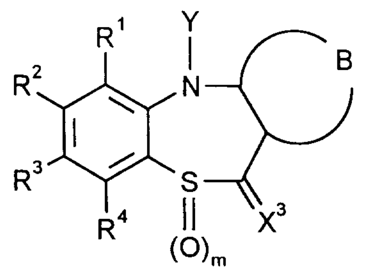

(V),

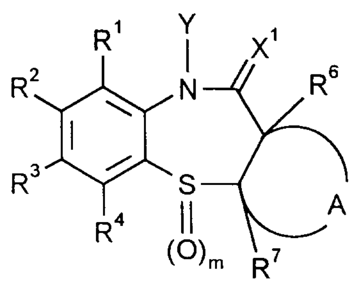

(VI) und

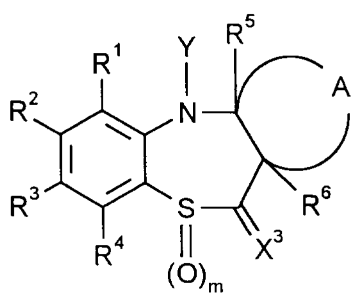

(VII),

worin

| | |
|---|---|
| $R^1$, $R^2$, $R^3$, $R^4$ | gleich oder verschieden sind und für Wasserstoff, Hydroxy, Amino, Cyano, Halogen, Nitro, Nitroso, Sulfonyl, Sulfoxyl, Tosyl, Mercapto, Carboxyl, Carboxyamid, Carbalkoxy, Dithiocarboxyl, Thiocarboxylamid, Dithiocarbalkoxy, gegebenenfalls substituiertes Alkyl, Aryl, Heteroaryl, Alkoxy, Aryloxy, Heteroaryloxy, Acyloxy, Alkylthio, Arylthio, Heteroarylthio, Acylthio, Acyl, Thioacyl oder Acylamino stehen oder untereinander einen oder mehrere gesättigte oder ungesättigte, gegebenenfalls substituierte isocyclische oder heterocyclische Kohlenstoffringe mit bis zu 8 C-Atomen bilden, |
| Y | Wasserstoff, gegebenenfalls substituiertes Alkyl, Aryl, Heteroaryl, Acyl, Thioacyl, Acyloxy, Arylamino oder Acylamino bedeutet, |

| | |
|---|---|
| $X^1$, $X^2$ oder $X^3$ | folgende Gruppierungen bedeutet: |

=O, =S, =N-Z, 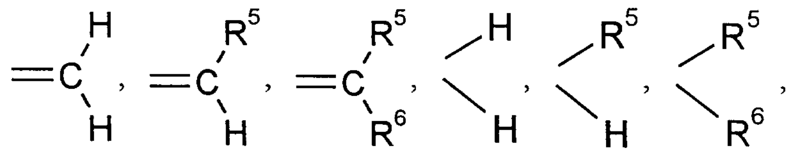

| | |
|---|---|
| $R^5$, $R^6$ und $R^7$ | gleich oder verschieden sind und die Bedeutung von $R^1$ bis $R^4$ besitzen, mit der Ausnahme, daß sie untereinander keinen cyclischen Ring bilden können, |
| Z | die Bedeutung von Y hat mit der Ausnahme, daß Z nicht gleich H sein kann, |
| A | die Anellierung eines gegebenenfalls substituierten gesättigten isocyclischen oder heterocyclischen Rings mit bis zu 8 C-Atomen bedeutet, |
| B | die Anellierung eines gegebenenfalls substituierten ungesättigten isocyclischen oder heterocyclischen Rings mit bis zu 8 C-Atomen bedeutet und |
| m | 0 oder 1 bedeutet, |

cyclische benzokondensierte Imine der Formel

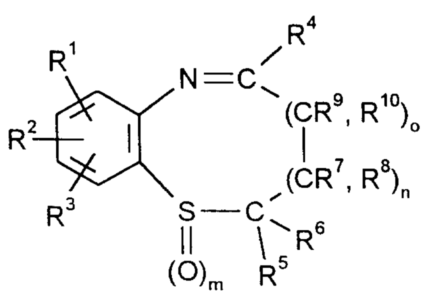

(VIII),

in der

| | |
|---|---|
| $R^1$ und $R^2$ | unabhängig voneinander Wasserstoff, Hydroxy, Amino, Cyano, Halogen, Nitro, Carboxyl, Halogenocarbonyl, Carboxyamid, Alkoxycarbonyl, Alkyl, Aryl, Alkoxy, Aryloxy, Acyloxy, Alkylthio, Arylthio, Acylthio, Acyl, Thioacyl oder Acylamino bedeuten, |
| $R^3$ | für Wasserstoff oder Chlor steht und weiterhin mit einem der Reste $R^1$ oder $R^2$ bei benachbarter Substitution und gemeinsam mit den substituierten C-Atomen einen anellierten gesättigten, ungesättigten oder aromatischen isocyclischen oder heterocyclischen 5-8-Ring bilden kann, |
| $R^4$ | Wasserstoff, Alkyl, Aryl, Halogen, Alkylthio, Arylthio, Alkoxy, Aryloxy, Amino, Hydrazino, Alkylhydrazino oder Phenylhydrazino bedeutet, |
| m, n und o | unabhängig voneinander den Wert 0 oder 1 annehmen können, |
| $R^5$, $R^7$ und $R^9$ | unabhängig voneinander Wasserstoff, Alkyl, Alkoxy, Phenyl, Acyloxy, Cyano, Halogen, Carboxyl, Alkoxycarbonyl, Phenoxy oder Acyl bedeuten und |

$R^6$, $R^8$ und $R^{10}$ unabhängig voneinander Wasserstoff, Alkyl oder Halogen bedeuten, wobei $R^5$ und $R^7$ oder $R^7$ und $R^9$ gemeinsam mit den substituierten C-Atomen einen gesättigten, ungesättigten oder aromatischen isocyclischen oder heterocyclischen 5-8-Ring darstellen können und wobei

weiterhin $R^6$ und $R^8$ oder $R^8$ und $R^{10}$ gemeinsam eine Doppelbindung bilden können und wobei weiterhin $R^5$ und $R^6$ gemeinsam doppelt gebundene Sauerstoff, Schwefel oder $R^{11}$-subsituierten Stickstoff darstellen können, wobei $R^{11}$ Alkyl, Aryl, Acyl, Alkylamino oder Arylamino bedeutet,
Benzo[f]-1,4-thiazepine der Formel

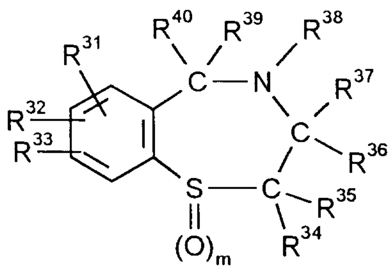

(IX),

in der

$R^{31}$ und $R^{32}$ unabhängig voneinander Wasserstoff, Hydroxy, Amino, Cyano, Halogen, Nitro, $C_1$-$C_8$-Alkyl, nicht substituiertes oder durch $R^{31}$ und $R^{32}$ substituiertes Phenyl (mit Ausnahme der erneuten Substitution durch $R^{31}$- und $R^{32}$-substituiertes Phenyl), $C_1$-$C_8$-Alkoxy, Phenoxy, $C_1$-$C_8$-Acyloxy, $C_1$-$C_8$-Acyl oder $C_1$-$C_8$-Alkoxycarbonyl bedeuten,

$R^{33}$ für Wasserstoff oder Chlor steht und weiterhin mit einem der Reste $R^{31}$ oder $R^{32}$ und gemeinsam mit den substituierten C-Atomen einen anellierten gesättigten, ungesättigten oder aromatischen isocyclischen oder heterocyclischen 5-8-Ring bilden kann,

$R^{34}$, $R^{36}$ und $R^{40}$ unabhängig voneinander Wasserstoff, $C_1$-$C_8$-Alkyl, nicht substituiertes oder durch $R^{31}$ und $R^{32}$ substituiertes Phenyl (mit Ausnahme der erneuten Substitution durch $R^{31}$- und $R^{32}$-substituiertes Phenyl), $C_1$-$C_8$-Acyl, $C_1$-$C_8$-Alkoxycarbonyl, Cyano, Halogen, Carboxyl, $C_1$-$C_8$-Alkoxy, $C_1$-$C_8$-Alkylthio, Phenylthio, Benzylthio, Phenoxy oder $C_1$-$C_8$-Acyloxy bedeuten,

$R^{35}$, $R^{37}$ und $R^{39}$ unabhängig voneinander Wasserstoff, $C_1$-$C_8$-Alkyl, Halogen, $C_1$-$C_8$-Alkoxy oder $C_1$-$C_8$-Alkylthio bedeuten,

$R^{38}$ Wasserstoff, $C_1$-$C_8$-Alkyl, nicht substituierte oder durch $R^{31}$- und $R^{32}$-substituiertes Phenyl (mit Ausnahme der erneuten Substitution durch $R^{31}$- und $R^{32}$-substituiertes Phenyl), $C_1$-$C_8$-Acyl, $C_1$-$C_8$-Thioacyl, Halogencarbonyl oder $C_1$-$C_8$-Alkoxycarbonyl bedeutet und

p für die Zahl 0 oder 1 steht,

wobei weiterhin
die Substituentenpaare $R^{34}$ und $R^{35}$, $R^{36}$ und $R^{37}$ sowie $R^{39}$ und $R^{40}$ unabhängig voneinander doppelt gebundenen Sauerstoff, Schwefel oder $R^{38}$-substituierten Stickstoff bedeuten können und wobei weiterhin
die Substituentenpaare $R^{35}$ und $R^{36}$ sowie $R^{38}$ und $R^{39}$ unabhängig voneinander eine Doppelbindung bilden können und wobei weiterhin
die Substituentenpaare $R^{34}$ und $R^{37}$ sowie $R^{38}$ und $R^{39}$ unabhängig voneinander 3- bis 5-gliedriges Alkylen bilden können, bei dem 1 oder 2 C-Atome durch Sauerstoff, Schwefel oder $R^{38}$-substituierten Stickstoff ersetzt sein können, und wobei weiterhin
$R^{40}$ auch die Bedeutung Hydrazino, $C_1$-$C_8$-Alkylhydrazino oder Phenyl-hydrazino

annehmen kann,
am exocyclischen N-Atom oxysubstituierte cyclische Amidine der Formel

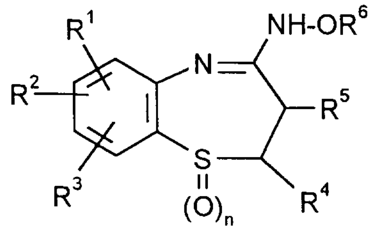

(X),

in der

| | |
|---|---|
| $R^1$ und $R^2$ | unabhängig voneinander Wasserstoff, Cyano, Halogen, Carboxyl, Alkoxycarbonyl, Alkyl, Aryl, Alkoxy, Aryloxy oder Acyl bedeuten, |
| $R^3$ | für Wasserstoff, Alkyl oder Chlor steht und weiterhin mit einem der Reste $R^1$ oder $R^2$ bei benachbarter Substitution und gemeinsam mit den substituierten C-Atomen einen anellierten gesättigten, ungesättigten oder aromatischen, isocyclischen oder heterocyclischen 5-8-Ring bilden kann, |
| $R^4$ und $R^5$ | unabhängig voneinander Wasserstoff, Alkyl, Aryl, Halogen, Alkoxy, Aryloxy, Acyl oder Acyloxy bedeuten oder gemeinsam mit den substituierten C-Atomen einen gesättigten oder ungesättigten, isocyclischen oder heterocyclischen 5-8-Ring bilden können, |
| $R^6$ | Wasserstoff, Alkyl, Aryl oder durch Alkyl oder Aryl substituiertes Silyl bedeutet und |
| n | den Wert 0 oder 1 annehmen kann, |

1,6-Benzo-thiazocine der Formel

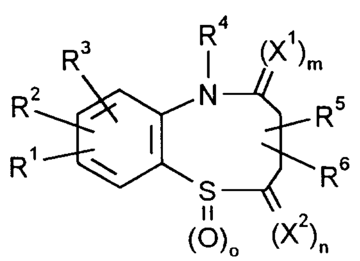

(XI),

in der

| | |
|---|---|
| $R^1$ und $R^2$ | unabhängig voneinander Wasserstoff, Hydroxy, Amino, Cyano, Halogen, Nitro, Alkylsulfonyl, Phenylsulfonyl, Alkylsulfoxyl, Phenylsulfoxyl, Tosyl, Mercapto, Carboxyl, Halogencarbonyl, Carboxyamid, Alkoxycarbonyl, Thiocarboxyamid, Alkyl, Aryl, Heteroaryl, Alkoxy, Aryloxy, Heteroaryloxy, Acyloxy, Alkylthio, Arylthio, Heteroarylthio, Acylthio, Acyl, Thioacyl oder Acylamino bedeuten, |
| $R^3$ | für Wasserstoff oder Chlor steht und weiterhin mit einem der Reste $R^1$ oder $R^2$ und gemeinsam mit den substituierten C-Atomen einen anellierten gesättigten, ungesättigten oder aromatischen isocyclischen oder heterocyclischen 5-8-Ring bilden kann, |

$R^4$ Wasserstoff, Alkyl, Aryl, Heteroaryl, Acyl, Thioacyl, Halogencarbonyl oder Alkoxycarbonyl bedeutet,

$X^1$ und $X^2$ unabhängig voneinander für doppelt gebundenen Sauerstoff, Schwefel oder $R^7$-substituierten Stickstoff stehen wobei $R^7$ den Bedeutungsumfang von $R^4$ mit Ausnahme von Wasserstoff hat,

m, n und o unabhängig voneinander den Wert 0 oder 1 annehmen können und

$R^5$ und $R^6$ unabhängig voneinander an einem oder an zwei der zwischen dem S- und dem N-Atom in 8-Ring befindlichen C-Atome stehen können, sofern diese C-Atome nicht durch $X^1$ bzw. $X^2$ besetzt sind, und den Bedeutungsumfang von $R^1$ bzw. $R^2$ haben, wobei bei benachbarter Substitution auch mit den substituierten C-Atomen ein gesättigter, ungesättigter oder aromatischer isocyclischer oder heterocyclischer 5-8-Ring gebildet werden kann und wobei weiterhin der Bedeutungsumfang des doppelt gebundenen Sauerstoffs oder Schwefels angenommen werden kann und

N-substituierte Phenothiazinderivate der Formel

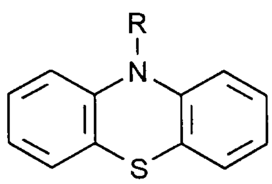 (XII),

in der

R ein Arylrest oder

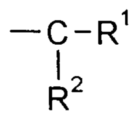

ist, wobei

$R^1$ =O, =S,

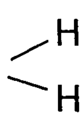 oder 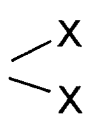

bedeutet und

X Br oder Cl ist und

$R^2$ einen Arylrest, Br, Cl oder den Rest $-CH_xX_y$ bedeutet, in dem

X Br oder Cl,

x einen Wert von 0 bis 2,

y einen Wert von 1 bis 3 bedeutet und
$x + y = 3$ ist oder
in der

R ein $CF_3$-$(CF_2)_n$-CO-Rest ist, bei dem

n für Null, 1 oder 2 steht.

Bevorzugt eingesetzt werden 1,4-Thiazepinderivate und Dibenzo-1,4-thiazinderivate, besonders bevorzugt Benzo[b]-1,4-thiazepinderivate und N-Perfluoracylsubstituierte Phenothiazine. Man kann in beiden Stufen des Verfahrens den gleichen Co-Katalysator, aber auch verschiedene Co-Katalysatoren einsetzen. Außerdem kann man für beide Stufen auch Gemische der genannten Co-Katalysatoren einsetzen.

In der ersten Reaktionsstufe kann man beispielsweise 0,005 bis 0,75 Gew.-% Co-Katalysator (bezogen auf 3-Fluortoluol) einsetzen. Vorzugsweise beträgt diese Menge 0,05 bis 0,5 Gew.-%. In die zweite Reaktionsstufe kann man beispielsweise weitere 0,1 bis 5 Gew.-% Co-Katalysator (bezogen auf 3-Fluortoluol) zufügen. Vorzugsweise beträgt diese zusätzliche Menge 0,3 bis 2 Gew.-%.

Man kann beide Reaktionsstufen des erfindungsgemäßen Verfahrens in Anwesenheit oder Abwesenheit von Lösungsmitteln durchführen. Als Lösungsmittel kommen z.B. halogenierte $C_1$-$C_4$-Kohlenwasserstoffe in Frage wie Tetrachlorkohlenstoff oder Dichlormethan sowie, insbesondere für die zweite Reaktionsstufe, auch längerkettige halogenierte aliphatische Kohlenwasserstoffe mit entsprechend höheren Siedepunkten. Vorzugsweise arbeitet man in beiden Reaktionsstufen ohne Lösungsmittelzusätze.

Die erste Reaktionsstufe kann man beispielsweise bei 0 bis 50°C durchführen und pro Mol 3-Fluortoluol z.B. 0,8 bis 1,1 Mol Chlor einleiten. Vorzugsweise arbeitet man in der ersten Reaktionsstufe bei 10 bis 30°C und leitet pro Mol 3-Fluortoluol 0,95 bis 1,01 Mol Chlor ein. Besonders bevorzugt arbeitet man bei Raumtemperatur und leitet pro Mol 3-Fluortoluol 1 Mol Chlor ein.

Die zweite Reaktionsstufe kann man beispielsweise bei 60 bis 120°C durchführen und pro Mol 3-Fluortoluol z.B. 0,8 bis 1,0 Mol Chlor einleiten. Vorzugsweise arbeitet man in dieser Reaktionsstufe bei 70 bis 100°C und leitet pro Mol 3-Fluortoluol 0,95 bis 0,98 Mol Chlor ein.

Der Druck bei der Durchführung der ersten und zweiten Reaktionsstufe des erfindungsgemäßen Verfahrens kann jeweils beispielsweise im Bereich 1 bis 10 bar liegen. Bevorzugt ist Normaldruck.

In beide Reaktionsstufen kann man das elementare Chlor gasförmig oder flüssig einleiten.

Das nach Beendigung der zweiten Reaktionsstufe vorliegende Reaktionsgemisch kann man z.B. aufarbeiten, indem man die Katalysatoren, z.B. durch Filtration, abtrennt und gegebenenfalls vorhandenen Chlorwasserstoff ausbläst. Man kann den im allgemeinen weniger wertvollen Friedel-Crafts-Katalysator auch durch eine ein- oder mehrfache Wäsche mit Wasser entfernen und dann das verbleibende Reaktionsgemisch destillativ aufarbeiten. Dabei verbleiben die wertvolleren Co-Katalysatoren im Destillationsrückstand und können in Form dieses Rückstands oder nach Aufarbeitung wieder eingesetzt werden.

Die Trennung der beiden im Reaktionsgemisch vorhandenen Isomeren (3-Fluor-4,6-dichlortoluol und 3-Fluor-2,6-dichlortoluol) ist nach an sich bekannten Methoden möglich, z.B. durch Destillation. Wenn das 3-Fluor-4,6-dichlortoluol verwendet werden soll, um daraus 3-Fluor-4,6-dichlorbenzoylchlorid herzustellen, dann kann man die Isomerentrennung auch erst nach der Seitenkettenchlorierung aus dem dann vorliegenden Benzalchlorid/Benzotrichlorid-Gemisch heraus durchführen.

Es ist ausgesprochen überraschend, daß das erfindungsgemäße Zweistufen-Verfahren Reaktionsgemische mit einem deutlich erhöhten Gehalt an 3-Fluor-4,6-dichlortoluol liefert als ein entsprechendes einstufiges Verfahren, bei dem die gesamte Chlorierung bei gleichen Reaktionsbedingungen und ohne Nachdosierung von Katalysatoren durchgeführt wird.

Das erfindungsgemäße Verfahren zur Herstellung von 3-Fluor-4,6-dichlortoluol hat eine Reihe von Vorteilen. So kann es in üblichen technischen Anlagen und mit gängigen Chemikalien in beliebig großen Mengen 3-Fluor-4,6-dichlortoluol liefern. Außerdem ist es technisch wesentlich einfacher als bekannte Verfahren und erfordert nicht den Umgang mit schwer handhabbaren Stoffen. Schließlich ist auch vorteilhaft, daß die wertvollen Co-Katalysatoren wiederverwendet werden können. Insgesamt wird mit dem erfindungsgemäßen Verfahren ein sehr günstiger Weg zur Herstellung eines wichtigen Zwischenprodukts für Wirkstoffe aus der Gruppe der Chinoloncarbonsäuren zur Verfügung gestellt.

Die nachstehenden Beispiele erläutern die Erfindung.

**Beispiele**

Alle Prozentangaben sind Gewichtsprozente.

**Beispiel 1**

In einem Chlorierbecher wurden 275 g 3-Fluortoluol, 0,55 g Eisen(III)-chlorid und 0,90 g N-Trifluoracetylphenothiazin vorgelegt und bei 20°C unter Rühren 178 g Chlor im Verlaufe von 6 Stunden gleichmäßig eingeleitet. Nach 15 Minuten Nachrührzeit wurden 1,53 g Eisen(III)-chlorid und 2,18 g N-Trifluoracetylphenothiazin zudosiert, auf 80°C erhitzt und bei dieser Temperatur unter Rühren weitere 179 g Chlor im Verlaufe von 6 Stunden gleichmäßig eingeleitet. Das erhaltene Produktgemisch wurde mittels GC analysiert. Es enthielt 1,3 % 3-Fluor-6-chlortoluol, 75,0 % 3-Fluor-4,6-dichlortoluol und 20,3 % 3-Fluor-2,6-dichlortoluol. Das ergibt eine Bildungsselektivität für 3-Fluor-4,6-dichlortoluol von 76 %.

**Beispiel 2**

Beispiel 1 wurde wiederholt, jedoch wurden in die 2. Stufe statt 2,18 g N-Trifluoracetylphenothiazin 0,30 g 2,3-Dihydroxybenzo-[b]-1,4-thiazepin-5-on eingesetzt. Das erhaltene Produktgemisch enthielt gemäß GC-Analyse 2,0 % 3-Fluor-6-chlortoluol, 72,0 % 3-Fluor-4,6-dichlortoluol und 22,1 % 3-Fluor-2,6-dichlortoluol, entsprechend einer Bildungsselektivität für 3-Fluor-4,6-dichlortoluol von 73,5 %.

**Beispiel 3** (zum Vergleich)

In einem Chlorierbecher wurden 275 g 3-Fluortoluol, 2,55 g Eisen(III)-chlorid und 2,90 g N-Trifluoracetylphenothiazin vorgelegt und bei 80°C 256 g Chlor im Verlaufe von 15 Stunden gleichmäßig eingeleitet. Das erhaltene Produktgemisch enthielt gemäß GC-Analyse 2,5 % 3-Fluor-6-chlortoluol. Die Bildungsselektivität für 3-Fluor-4,6-dichlortoluol betrug nur 65,3 %.

**Beispiel 4** (zum Vergleich)

Beispiel 3 wurde wiederholt, jedoch wurden statt 2,90 g N-Trifluoracetylphenothiazin 0,60 g 2,3-Dihydrobenzo-(b]-1,4-thiazepin-5-on eingesetzt. Das erhaltene Produktgemisch enthielt gemäß GC-Analyse 10,3 % 3-Fluor-6-chlortoluol. Die Bildungsselektivität für 3-Fluor-4,6-dichlortoluol betrug nur 59,8 %.

## Patentansprüche

1. Verfahren zur Herstellung von 3-Fluor-4,6-dichlortoluol, dadurch gekennzeichnet, daß man 3-Fluortoluol in Gegenwart eines Friedel-Crafts-Katalysators und eines heterocyclischen Co-Katalysators zunächst bei niedriger Temperatur chloriert und ein Gemisch erhält, das 3-Fluor-4-chlorbenzol und 3-Fluor-6-chlorbenzol enthält, und anschließend dieses Gemisch ohne Zwischenisolierung nach Zusatz von weiterem Friedel-Crafts-Katalysator und weiterem heterocyclischen Co-Katalysator bei höherer Temperatur zu 3-Fluor-4,6-dichlortoluol weiterchloriert.

2. Verfahren nach Anspruch 1, dadurch gekennzeichnet, daß man als Friedel-Crafts-Katalysatoren Eisen(III)-chlorid, Antimon(III)-chlorid, Antimon(V)-chlorid oder Aluminiumchlorid einsetzt.

3. Verfahren nach Ansprüchen 1 und 2, dadurch gekennzeichnet, daß man in die erste Reaktionsstufe 0,05 bis 1 Gew.-% Friedel-Crafts-Katalysator (bezogen auf 3-Fluortoluol) einsetzt und in die zweite Reaktinsstufe weitere 0,1 bis 5 Gew.-% Friedel-Crafts-Katalysator (bezogen auf 3-Fluortoluol) zufügt.

4. Verfahren nach Ansprüchen 1 bis 3, dadurch gekennzeichnet, daß man als Co-Katalysatoren Heterocyclen einsetzt, die gleichzeitig N- und S-Atome enthalten und den Klassen der Thiazine, Thiazepine oder Thiazocine angehören.

5. Verfahren nach Ansprüchen 1 bis 4, dadurch gekennzeichnet, daß als Co-Katalysatoren Benzo[b]-1,4-thiazepinderivate und/oder N-Perfluoracylsubstituierte Phenothiazine eingesetzt werden.

6. Verfahren nach Ansprüchen 1 bis 5, dadurch gekennzeichnet, daß als Co-Katalysatoren N-Trifluoracetylphenothiazin und/oder 2,3-Dihydrobenzo-[b]-1,4-thiazepin-5-on eingesetzt werden.

7. Verfahren nach Ansprüchen 1 bis 6, dadurch gekennzeichnet, daß man in die erste Reaktionsstufe 0,005 bis 0,75 Gew.-% Co-Katalysator (bezogen auf 3-Fluortoluol) einsetzt und in die zweite Reaktionsstufe weitere 0,1 bis 5 Gew.-% Co-Katalysator (bezogen auf 3-Fluortoluol) zufügt.

8. Verfahren nach Ansprüchen 1 bis 7, dadurch gekennzeichnet, daß man die erste Reaktionsstufe bei 0 bis 50°C und die zweite Reaktionsstufe bei 60 bis 120°C durchführt.

9. Verfahren nach Ansprüchen 1 bis 8, dadurch gekennzeichnet, daß man in die erste Reaktionsstufe 0,8 bis 1,1 Mol Chlor pro Mol 3-Fluortoluol und in die zweite Reaktionsstufe 0,8 bis 1,0 Mol Chlor pro Mol 3-Fluortoluol einleitet.

10. Verfahren nach Ansprüchen 1 bis 9, dadurch gekennzeichnet, daß man das nach Beendigung der zweiten Reaktionsstufe vorliegende Reaktionsgemisch destillativ aufarbeitet und die dabei im Destillationsrückstand anfallenden Co-Katalysatoren wieder einsetzt.

Europäisches Patentamt

# EUROPÄISCHER RECHERCHENBERICHT

Nummer der Anmeldung
EP 95 11 9332

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| Y | EP-A-0 114 604 (BAYER AG)<br>* Ansprüche *<br>--- | 1 | C07C17/12<br>C07C25/13 |
| Y | EP-A-0 442 115 (BAYER AG)<br>* Ansprüche *<br>--- | 1 | |
| Y | EP-A-0 368 063 (BAYER AG)<br>* Ansprüche *<br>--- | 1 | |
| Y | EP-A-0 340 581 (BAYER AG)<br>* Ansprüche *<br>--- | 1 | |
| Y | EP-A-0 292 824 (BAYER AG)<br>* Ansprüche *<br>--- | 1 | |
| Y | FR-A-2 545 004 (PCUK PRODIUTS CHIMIQUES UGINE KUHLMANN)<br>* Ansprüche *<br>--- | 1 | |
| P,A | EP-A-0 657 407 (BAYER AG)<br>* das ganze Dokument *<br>----- | 1 | |

RECHERCHIERTE SACHGEBIETE (Int.Cl.6)
C07C

Der vorliegende Recherchenbericht wurde für alle Patentansprüche erstellt

| Recherchenort | Abschlußdatum der Recherche | Prüfer |
|---|---|---|
| DEN HAAG | 25.März 1996 | Bonnevalle, E |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
..........................................
& : Mitglied der gleichen Patentfamilie, übereinstimmendes Dokument

EPO FORM 1503 03.82 (P04C03)